# EUROPEAN PATENT SPECIFICATION

(11) **EP 3 454 663 B1**
(45) Date of publication and mention of the grant of the patent: **11.12.2024**
(21) Application number: 16727830.8
(22) Date of filing: 12.05.2016
(51) Int. Cl.: A23D 9/013, A23D 7/01

(54) **LOW MOLECULAR WEIGHT ORGANIC GELATORS OF VEGETABLE OIL**
NIEDERMOLEKULARE ORGANISCHE GELATOREN VON PFLANZENÖL
GÉLIFIANTS ORGANIQUES D'HUILE VÉGÉTALE DE FAIBLE POIDS MOLÉCULAIRE

(43) Date of publication of application: 20.03.2019
(73) Proprietor: Rudjer Boskovic Institute, 10000 Zagreb (HR)
(72) Inventor: SIJAKOVIC VUJICIC, Natasa, 10360 Sesvete (HR)
(74) Representative: Vukmir, Mladen
(86) International application number: PCT/HR2016/000016
(87) International publication number: WO 2017/194980

(56) References cited:
- WO-A2-2008/102127
- US-A1- 2011 207 813
- US-A1- 2011 224 124
- JOKIC M ET AL: "A NOVEL TYPE OF SMALL ORGANIC GELATORS: BIS(AMINO ACID) OXALYL AMIDES", JOURNAL OF THE CHEMICAL SOCIETY, CHEMICAL COMMUNICATIONS, ROYAL SOCIETY OF CHEMISTRY, GB, no. 17, 1 January 1995 (1995-01-01), pages 1723/1724, XP009029078, ISSN: 0022-4936, DOI: 10.1039/C39950001723
- MAKAREVIC J ET AL: "Bis(amino acid) oxalyl amides as ambidextrous gelators of water and organic solvents: supramolecular gels with temperature dependent assembly/dissolution equilibrium", CHEMISTRY - A EUROPEAN JOURNAL, WILEY - V C H VERLAG GMBH & CO. KGAA, DE, vol. 7, no. 15, 1 January 2001 (2001-01-01), pages 3328 - 3341, XP002276456, ISSN: 0947-6539, DOI: 10.1002/1521-3765(20010803)7:15<3328::AID-CHEM3328>3.0.CO;2-C
- LUO X ET AL: "Self-assembled organogels formed by mono-chain L-alanine derivatives", CHEMICAL COMMUNICATIONS - CHEMCOM,, no. 17, 7 September 2001 (2001-09-07), pages 1556 - 1557, XP002220130, ISSN: 1359-7345, DOI: 10.1039/B104428C
- RAHUL R. MAHIRE ET AL: "Fabrication of organogels achieved by prodrug-based organogelators of ketoprofen", RSC ADVANCES: AN INTERNATIONAL JOURNAL TO FURTHER THE CHEMICAL SCIENCES, vol. 4, no. 63, 11 July 2014 (2014-07-11), GB, pages 33286, XP055318502, ISSN: 2046-2069, DOI: 10.1039/C4RA03688C

## Description

### FIELD OF THE INVENTION

The invention relates to the composition with gelling properties comprising low molecular weight gelators of Formula (I) and a vegetable oil. The invention further relates to the use of such composition in the food, cosmetic or pharmaceutical industry.

### BACKGROUND OF THE INVENTION

The World Health Organization (WHO) indicates the importance of controlling the intake of high levels of saturated fatty acids compared to reduced intake of mono- and poly-unsaturated fatty acids. However, saturated fatty acids are indispensable in food products, especially margarine, spreads, meat products and confectionery products, where they control key physical properties of the product (solid state, texture, spreadability and taste). Therefore, in recent years the food industry searches for a new effective way of structuring edible oils, to prevent potential health issues.

The conventional approach for structuring oil that is currently used in the food industries relies on the use of crystalline triacylglycerols (TAGs). These high melting TAGs are rich in saturated or even trans- fatty acids and the nutritional profile of this kind of structured oil systems is unhealthy. Also, structuring with this approach demands higher fractions of TAGs (till 20 wt %) compared with alternative methods. Alternative approaches that are based on the use of non-TAG structurants are nowadays explored to achieve efficient structuring of oil at comparatively lower concentrations.

It is necessary to distinguish different approaches of oil structuring through usage of low molecular weight organic compounds, polymeric compounds or inorganic compounds. Also, structuring of oil is possible through formation of network of crystalline particles, polymeric strands and self-assembled gel fibers.

As reviewed in the papers of Patel and co-workers (summarized in book Ed. Richard W. Hartel), Alternative Routes to Oil Structuring, Springer, 2015) many different approaches of oil structuring have been explored.

The initial studies dealt mostly with the use of lipidic additives (such as long chain fatty acids, fatty alcohols, dicarboxylic acids, wax esters, hydroxylated fatty acids, natural waxes and partial glycerides) as structuring agents of edible oil via direct dispersion at elevated temperatures followed by cooling. One example in this field concerns about lipid composition with structuring agent comprises at least 10 wt % of diacyclglycerols having a very long chain saturated fatty acid residue (WO2014/184118A1).

There are also few publications demonstrating two-component mixtures that showed synergistic gelling functionality such as stearic acid + stearyl alcohol, β -sitosterol + oryzanol, lecithin + sorbitan tristearate and mixtures of ceramides (E. D. Co and A. G. Marangoni, J. Am. Oil. Chem. Soc. 2012, 89, 749-780). Thereafter, a polymers such as modified cellulose (ethyl cellulose, EC) and proteins were also studied to extend the structuring principle beyond the crystalline network formation as seen with the lipid-based materials. In recent years, research on hydrophilic polymer-based gels, resin wax (shellac wax) gels and inorganic particle-based gels have also been published (J. Am. Oil. Chem. Soc. 2015, 92, 801-811; Eur. J. Lipid Sci. Technol., 2015, 117, 1772-1781).

The natural waxes are approved as indirect additives, since there are regulatory concerns which need to be addressed. The major problem is tendency of waxes to post crystallization processes and instability of wax-based gels for longer storage period. Polymers such as cellulose derivatives, proteins and other hydrophilic polysaccharides are less feasible for scale-up, since there are additional processing steps required for gelation of oil (high temperature treatment, lyophilisation).

There have been identified many potential structurants of oil, but there is still a need to find a food-grade oleogelator that is economical, efficient at low concentration, tolerant to processing conditions, compatible with the final composition of a product and primarily having a potent thixotropic property.

Structuring of oil through gelation phenomenon can serve as replacement of solid fats in both water-free (shortenings and chocolates) and water-containing (margarine, spreads and cooked meat products) products.

The main functional role of gelator agents in food formulation would be to provide structure and stabilization of the final product, possibility for controlled delivery of nutraceuticals and controlling of oil mobility and migration (allowing stability to chocolate products).

There has been a great study on low-molecular-weight organic gelators (LMWOG) over the past decades, because of its academic interests and potential applications to cosmetics, foods, medical and pharmaceutical, photonic and electronic devices.

Through intensive research during the last two decades, more than 1000 structurally different low-molecular weight organic gelators (LMWOG) were shown to exhibit gelling ability toward various organic solvents and water. Although many low-molecular-weight gelators have been discovered and a few low-molecular-weight gelators have been used in cosmetics and commodities, their market shares are small, compared with polymer gelators.

In pharmaceutical industry, the LMWOG were explored mostly as hydrogelators for controlled release drug delivery. There are few examples of organogelators in pharmaceutical industry where LMWOG were explored for controlled release of bioactive substances (WO2009095485, US2005031650A1). Among them, US 2011/207813 A1 describes pharmaceutical composition with gelling properties containing a tyrosine derivative wherein WO 2008/102127 relates to pyridyl derivatives with gelling properties in water and organic solvents. Use of organogels in cosmetics is described in US2003091520.

Gel fibers, usually of micrometres scale lengths and nanometres scale diameters, are formed in solution through unidirectional self-assembly of gelator molecules. Such gels consist of a large amount of solvent and a very small amount of gelator molecules. The solvent is entrapped within the 3D network of entangled nanosize fibrous gelator aggregates. Due to the weak noncovalent interactions (hydrogen bonding, *π*-*π* stacking, van der Waals interactions and electrostatic interactions) that stabilize aggregates, most of the gels exhibit thermoreversible gel-to-sol transitions. An overview of the self-assembly of organogelators is presented in the book Molecular Gels: Structure and Dynamics, Editor: Richard G Weiss, Royal Society of Chemistry, 2018 and thousands of papers published in the last 25 years, for example paper of Luo X at all, Chem. Comm. 2001, 17, 1556-1557 (related to self-assembled organogels formed by mono-chain L-alanine derivatives).

The precise relationship between the gelator structure (constitution, configuration, and conformation), the properties of a solvent used to be gelled, and the motif of supramolecular organization within the gel fibers still remains to be discovered. Even very small variations of gelator constitution and changes of configuration can tremendously influence the gelation properties. Gelator stereochemistry has a noticeable influence on the gelation properties. For chiral gelators, both enantiomers have equal gelation properties, but symmetrical meso-diastereoisomers lack any gelation. In most cases, racemates are less efficient gelators than pure enantiomers, and sometimes lack any gelation ability. However, in the case of the present invention, some racemates also showed tremendous gelation capability.

A thixotropic supramolecular gel, which repeatedly undergoes the gel-to-sol transition by shearing and then sol-to-gel transition by standing, is a promising material. In spite of the many needs from industrial fields, it is very difficult to prepare such a gel. Although some thixotropic supramolecular gels have been reported, thixotropic supramolecular gels are serendipitously discovered with an amount less than a 1% in total.

The compounds of the present invention are described as organogelators for different organic solvents and water (Čaplar at al., *Chem. Eur. J.* 2010, 16, 3066 - 3082; Čaplar at al., *Eur. J. Org. Chem.* 2004, 4048-4059; Makarević at al., *Chem. Eur. J.* 2001, 7 (15), 3328 - 3341; Šijaković Vujičić at al. *Chem. Eur. J.* 2013, 19, 8558 - 8572). They have never been examined to gel vegetable oils or similar mixtures before and they have never before showed thixotropic behaviour in examined organic solvents and water. It is now surprisingly found that the compounds of the present invention may be used as superorganogelators of different vegetable oils. Additionally the organogelators of present invention showed self-healing (thixotropic) properties in vegetable oils. Also, they can be classified as superorganogelators of vegetable oil since they have ability to form gels in oil till concentration 0.02 wt%. The powerful applicability of oil gelators is envisaged in food (specifically in oil and meat industry), cosmetic and pharmaceutical industry.

### SUMMARY OF THE INVENTION

The present invention relates to the composition with gelling properties comprising a compound of Formula (I) or a salt thereof 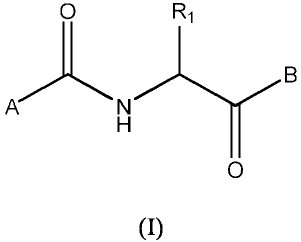 and a vegetable oil;
wherein A is selected from:
   i) -(CH₂)ₘ-CH₃,
   ii) -CO-NH-CH(R¹)-CO-R² and
   iii) -CO-NH-(CH₂)ₚ-NH-CO-CO-NH-CH(R¹)-CO-R²
B is selected from:
   i) -NH-(CH₂)ₙ-CO-R² and
   ii) -R².
R¹ is H, -C₁-C₄ alkyl, phenyl or -CH₂Ph,
R² is -OH, -NH₂ or -OR³;
R³ is -C₁-C₄ alkyl or benzyl;
*m* is an integer from 1-34,
*n* is an integer from 1-22
and *p* is an integer from 1-12;
provided that when A is -(CH₂)ₘ-CH₃ then B is -NH-(CH₂)ₙ-CO-R²
and when B is -NH-(CH₂)ₙ-CO-R² then A is -(CH₂)ₘ-CH₃.

The present invention further relate to the use of the composition comprising a compound of formula (I) or a salt thereof and the oil, in food, cosmetic or pharmaceutical industry.

### BRIEF DESCRIPTION OF THE FIGURES

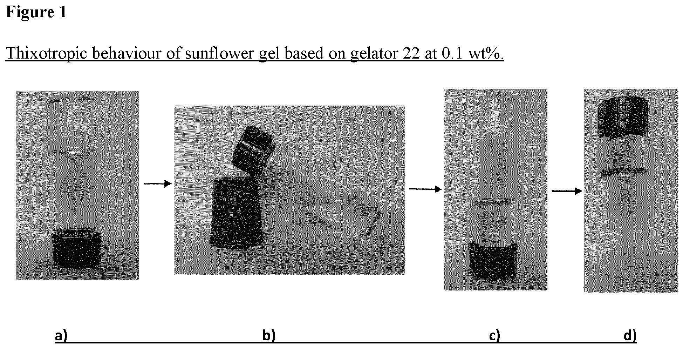

Figure 1 shows thixotropic behaviour of sunflower gel based on gelator 22 at 0.1 wt%. (a) Gel formed from hot solution upon cooling; (b, c) Low-viscosity fluid formed by vigorous hand shaking; (d) Gel reformed after standing for 5 min at room temperature.

### DETAILED DESCRIPTION OF THE INVENTION

The following abbreviations are used in the text: DCC for N,N'-dicyclohexylcarbodiimide, Et₃N for thriethylamine, DMAP for 4-Dimethylaminopyridine, Boc for tert-butyloxycarbonyl protecting group, Ph for phenyl.

The term "C₁-C₄ alkyl" as used herein, refers to a saturated, straight or branched-chain hydrocarbon radical containing between one and four carbon atoms. Examples of "C₁-C₄ alky" radicals include: methyl, ethyl, n-propyl, isopropyl, n-butyl, isobutyl, *sec*-butyl and *tert*-butyl.

Organogelators or gelling agents represent molecules capable to self-assemble in different solvents into three-dimensional nano-network of self-assembled fibers through highly specific noncovalent interactions such as hydrogen bonding, van der Waals, *p*-stacking, electrostatic and charge-transfer interactions.

Organogels represent mixture of gel fibers present in solid state and liquid phase entrapped in pores between self-assembled fibers.

"Organogel of the present invention" or "organogel of the invention" as used herein refers to a composition comprising a compound of formula (1) or a salt thereof and oil as liquid phase structured into gel state.

"Vegetable oils" as used herein refers to edible oils or base cosmetic oils.

Edible oils are selected from palm oil, sunflower oil, olive oil, soybean oil, linseed oil, rapeseed oil, corn oil, pumpkin seed oil, sesame oil, safflower oil, castor, peanut oil and the like.

The cosmetic base oils are selected from sweet almond oil, linseed oil, grape seed oil, avocado oil, apricot oil, olive oil, sesame oil, rapeseed oil, sunflower oil, jojoba oil, castor oil, borage seed oil, argan oil, avocado oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil and the like.

"A composition according to present invention" or "a composition according to the invention" as used herein relates to the composition comprising the compound of formula (I) or a salt thereof and a vegetable oil.

"A compound of the present invention" or "a compound of the invention" as used herein relates to the compound of formula (I) or a salt thereof.

The term "thixotropy" as used herein refers to the property of certain gels that are thick (viscous) under normal conditions, but flow (become thin, less viscous) over time when shaken, agitated, or otherwise stressed and then take a fixed time to return to a more viscous, gel state.

Low molecular weight organic gelators are extremely sensitive to mechanical stress and these systems irreversibly expel solvent from their gel network when subjected to flow. On removal of the external force, these systems lose its original elastic properties. On the other hand, thixotropic gels can disintegrate in solution under an external mechanical stress and can regain their elastic properties upon removal of the stress. This operation can be carried out for an infinite number of cycles. Thixotropic LMOGs represent a unique class of dynamic self-assembled supramolecular systems.

Organogels have many different functionalities in food products, including restriction of oil mobility and stabilization of emulsions, especially in case were water droplets are entrapped inside the oleogel network.

An emulsion is termed an oil/water (o/w) emulsion if the dispersed phase is a liquid (an oil) and the continuous phase is water or an aqueous solution and is termed water/oil (w/o) if the dispersed phase is water or an aqueous solution and the continuous phase is an organic liquid (an oil).

Disclosed are compounds of formula (I) or a salts thereof as organogelators of vegetable oil.

In one aspect the present disclosure relates to the use of a compound of formula (I) or a salt thereof as an organogelator of vegetable oil.

The present invention relates to a composition comprising a compound of formula (I) or a salt thereof and a vegetable oil.

In one aspect suitable salt are base addition salts selected from sodium, potassium, calcium, and magnesium salt of compound of formula (I). In further aspect a salt is sodium salt of compound of formula (I).

In another aspect suitable salts are base addition salts selected from ammonium, or alkyl amonium salt of compound of formula (I). Examples of alkyl ammonium salt are methyl, ethyl, propyl, *iso*-propyl, *n*-butyl ammonium, tetramethylammonium, tetraethylammonium, tetrabutylammonium, ethylenediammonium salt or the like.

With regard to stereoisomers, the compounds of Formula (I) may have one or more asymmetric carbon atom. Thus, the compounds of Formula (I) may occur as individual enantiomers, diastereoisomers or mixtures thereof. All such isomeric forms are included within the present invention, including mixtures thereof.

In the following description, the groups A, B, R¹, R², R³, *m, n* and *p* have the meaning as defined for the compounds of Formula (I) unless otherwise stated.

In one aspect the present invention relates to the composition comprising a compound of formula (I) or a salt thereof and a vegetable oil wherein A is -(CH₂)ₘ-CH₃ and B is -NH-(CH₂)ₙ-CO-R². In further aspect *m* is an integer from 5-20. In yet further aspect *n* is an integer from 1-10. In yet further aspect R² is OH, -OMe or -NH₂. In yet further aspect R¹ is -Ph, and R² is -OH. In another aspect A is -(CH₂)ₘ-CH₃ , B is -NH-(CH₂)ₙ-CO-R², *m* and *n* are each 10, wherein R¹ is -Ph, - CH₂Ph or -CH(CH₃)₂ and R² is -OH.

In one aspect the present invention relates to the composition comprising a compound of formula (I) or a salt thereof and a vegetable oil wherein A is -(CH₂)ₘ-CH₃, B is -NH-(CH₂)ₙ-CO-R² and sum of *n* and *m* is between 18 and 22. In further aspect sum of *n* and *m* is 20.

In one aspect the present invention relates to the composition comprising a compound of formula (I) or a salt thereof and a vegetable oil wherein A is -CO-NH-CH(R¹)-CO-R² and B is R². In further aspect R¹ is -CH₂Ph, -CH₂CH(CH₃)₂ or -CH(CH₃)₂ and R² is -OH, -OCH₃ or -NH₂. In yet further aspect R¹ is -CH₂Ph, -CH₂CH(CH₃)₂ or -CH(CH₃)₂ and R² is -OH or -NH₂.

In one aspect the present invention relates to the composition comprising a compound of formula (I) or a salt thereof and a vegetable oil wherein A is -CO-NH-(CH₂)ₚ-NH-CO-CO-NH-CH(R¹)-CO-R² and B is R². In further aspect *p* is an integer from 4-10. In yet further aspect *p* is 6 - 9 wherein R¹ is -CH₂CH(CH₃)₂, and R² is -OCH₃, -OH or -NH₂. In yet further aspect p is 6 or 9 wherein R¹ is -CH₂CH(CH₃)₂, and R² is -OCH₃ or -OH.

The present invention relates to the composition comprising a compound of formula (I) or a salts thereof and a vegetable oil.

In one aspect the vegetable oil is edible oil.

In one further aspect the edible oil is selected from palm oil, sunflower oil, olive oil, soybean oil, linseed oil, rapeseed oil, corn oil, pumpkin seed oil, sesame oil, safflower oil, castor, peanut oil and combination thereof. In yet further aspect the edible oil is selected from sunflower oil, olive oil, soybean oil, rapeseed oil, palm oil, and combination thereof. In yet further aspect the edible oil is selected from sunflower oil, olive oil, soybean oil and combination thereof.

In one further aspect the present invention relates to the composition comprising a compound of formula (I) or a salts thereof and a vegetable cosmetic base oils. In further aspect the cosmetic base oil is selected from sweet almond oil, linseed oil, grape seed oil, avocado oil, apricot oil, olive oil, sesame oil, rapeseed oil, sunflower oil, jojoba oil, castor oil, borage seed oil, argan oil, avocado oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil or combination thereof. In yet further aspect the cosmetic base oil is selected from almond oil, avocado oil, jojoba oil, coconut oil, rice bran oil, peanut oil.

In one aspect the present invention relates to the composition comprising a compound of formula (I) or a salts thereof and a vegetable oil wherein the compound of formula (I) or a salts thereof is present in the composition in the concentration at which fluid motion of the oil stops.

In one aspect the present invention relates to the composition comprising a compound of formula (I) or a salts thereof and a vegetable oil wherein the compound of formula (I) or a salts thereof is present in the composition in the concentration at which gel is forming.

In one aspect the compound of formula (I) or a salts thereof is present in the composition at a concentration of about 20% or less, on a weight/weight basis. In another aspect, the compound of formula (I) or a salts thereof is present in the composition at a concentration of about 10%, on a weight/weight basis. In yet another aspect, the compound of formula (I) or a salts thereof is present in the composition at a concentration of about 5% or less, on a weight/weight basis. In another aspect, compound of formula (I) or a salts thereof is present in the composition at a concentration of about 2%, on a weight/weight basis. In yet another aspect, the compound of formula (I) or a salts thereof is present in the composition at a concentration of about 2% or less, on a weight/weight basis. In yet another aspect, the compound of formula (I) or a salts thereof is present in the composition at a concentration of about 0.5% or less, on a weight/weight basis. In yet another aspect, the compound of formula (I) or a salts thereof is present in the composition at a concentration of about 0.05% or less, on a weight/weight basis.

In one aspect, the compound of formula (I) or a salts thereof is present in the composition in the concentration of about 0.02 to about 10 wt % relative to the total weight of the composition.

In one aspect the present invention relates to the composition comprising a compound of formula (I) or a salts thereof, a vegetable oil and water. In further aspect the composition is in the form of a gelled water-in-oil emulsion or gelled oil in water emulsion.

In one aspect, the composition according to present invention forms an organogel.

In another aspect, the organogel recovers at least about 80% within less than about 10 minute, preferably within less than about 5 minute, and more preferably within less than about 1 minute after the exposure to destructive shear. In still another embodiment, the organogel recovers at least about 90% within less than about 10 minute, preferably within less than about 5 minute, and more preferably within less than about 1 minute after the exposure to destructive shear. In a further aspect, the organogel recovers at least about 95% within less than about 10 minute, preferably within less than about 5 minute, and more preferably within less than about 1 minute after the exposure to destructive shear.

In a still further aspect, the organogel recovers at least about 99% within less than about 10 minute, preferably within less than about 5 minute, and more preferably within less than about 1 minute after the exposure to destructive shear.

In one aspect, the composition according to present invention may be used in the pharmaceutical industry as vector/carriers for active substances.

In one aspect the present invention relates to the composition comprising a compound of formula (I) or a salt thereof, edible oil and the active pharmaceutical substance. In further aspect active pharmaceutical substance are provided for a sustained release.

In one aspect the present invention relates to the composition comprising a compound of formula (I) or a salts thereof, edible oil and the nutraceuticals substance wherein the nutraceuticals substance refers to dietary supplement such as vitamins, minerals, fatty acids, amino acids, proteins, herbal medicine etc.

In one aspect the invention relates to a composition comprising a compound of formula (I) or a salts thereof, edible oil and a food. In further aspect food is selected from shortenings and chocolates. In yet further aspect food is selected from margarine, spreads and cooked meat products.

In another aspect, the invention relates to a cosmetic composition comprising at least one cosmetically acceptable ingredient, a compound of formula (I) or a salts thereof and base cosmetic oil.

In one aspect, the invention relates to a composition usable as a cleaning tool in cultural heritage conservation comprising a compound of formula (I) or a salts thereof and oil.

In one aspect, the invention relates to a consumer product comprising a compound of formula (I) or a salts thereof and a vegetable oil. In further aspect consumer product is lubricant.

In one aspect the invention covers process for preparing the composition of the present invention comprising the following steps:
(a) mixing the compounds of formula (I) or a salt thereof and the vegetable oil;
(b) heating the mixture obtained in step (a) to a temperature until compound of formula (I) is completely dissolved;
(c) cooling the mixture obtained in step (b) to room temperature or below.

In one aspect the invention covers process for preparing the composition of the present invention comprising the following steps:
(a) mixing the food, pharmaceutically active ingredient, nutraceuticals or a cosmetic ingredient as solid or solubilised component in oil or water and the vegetable oil;
(b) heating the mixture obtained in step (a) to a temperature until compound of formula (I) is completely dissolved;
(c) adding and mixing the compounds of formula (I) or a salt thereof solubilised in oil during step (a) or step (b)
(d) cooling the mixture obtained in step (b) to room temperature or below.

In one aspect the invention covers process for preparing the composition of the present invention comprising the following steps:
(a) mixing the compounds of formula (I) or a salt thereof and the vegetable oil;
(b) heating the mixture obtained in step (a) to a temperature until compound of formula (I) is completely dissolved;
optionally (c) mixing the food, pharmaceutically active ingredient, nutraceuticals or a cosmetic ingredient as solid or solubilised component in oil or water into the mixture during the step (b); (d) cooling the mixture obtained in step (b) to room temperature or below.

Further alternatively, a food, a pharmaceutically active ingredient, a nutraceuticals or a cosmetic ingredient as solid or solubilised component in oil or water may be added to the mixture after or during step (d).

In one aspect the invention covers process for preparing the composition of the present invention comprising the following steps:
(a) mixing the compounds of formula (I) or a salt thereof and the vegetable oil; and optionally a food, pharmaceutically active ingredient, nutraceuticals or a cosmetic ingredient;
(b) heating the mixture obtained in step (a) to a temperature until compound of formula (I) is completely dissolved;
   optionally (c) heating of water soluble ingredients in a water phase;
(d) mixing of heated oil phase obtained in (b) and water phase obtained in (c);
(e) cooling the mixture obtained in step (d) to room temperature or below.

### Method of preparation:

Compounds of Formula (I) and salts thereof may be prepared by the general methods outlined hereinafter.

Suitable addition salt of compounds of Formula (I) may be prepared starting from corresponding free acid (R₂ is -OH) by the use of an equivalent quantity of suitable base.

Specifically, for the preparation of sodium salt, an equivalent quantity of 1M aqueous NaOH may be used. For the preparation of ammonium salt, equivalent quantity of corresponding amine may be used.

Compounds of Formula (I) wherein A is -(CH₂)ₘ-CH₃ and B is -NH-(CH₂)ₙ-CO-R² and R² is -OH may be prepared by saponification of corresponding ester (R₂ is -OCH₃) with LiOH in methanol/dichloromethane solution for optically active derivatives or with KOH or NaOH in methanol for racemic ones followed by acidification.

Compound of formula (I) wherein A is -(CH₂)ₘ-CH₃ and B is -NH-(CH₂)ₙ-CO-R² and R² is - NH₂ may be prepared starting from corresponding compound of formula (I) wherein R² is - OCH₃ by amonolysis in conc. NH₃/MeOH for 5-10 days at 0 °C.

Compounds of Formula (I) wherein A is -(CH₂)ₘ-CH₃ and B is -NH-(CH₂)ₙ-CO-R² wherein n is 2-22 R² is -OR³, may be prepared by reaction of acyl chloride of formula (II)

CH₃-(CH₂)ₘ-COCl (II)

with the compound of formula (III)

R²-CO-(CH₂)ₙ-NH-CO-CH(R¹)-NH₂ (III)

wherein n is 2-22 and R² is -OR³; in dry CH₂Cl₂ in the presence of Et₃N.

Compound of formula (III) wherein n is 2 - 22 may be prepared starting with corresponding amino acid of formula (IV)

NH₂-CH(R¹)-COOH (IV)

wherein amino group is protected for example with Boc protecting group, by condensation with the compound of formula (V)

NH₂(CH₂)ₙCOOR³ (V)

in the presence of DCC, Et₃N and DMAP in the aprotic solvent such as CH₂Cl₂ at room temperature followed by deprotection of amino protected group.

Reaction of condensation of compound of formula (IV) and (V) may also be carried out under activation by Ph₃P, in CCl₄/MeCN as solvent in the presence of Et₃N.

Alternatively, compound of formula (III) may be prepared starting with amino acid of formula (IV) wherein amino group is *Boc* protected and wherein carboxy group is activated with succinimide ester by coupling with compound of formula (V) in the presence of Et₃N in dry dioxane.

Compound of formula (V) are commercially available in case R³ is Me, *tert*-buthyl or benzyl, or are easily prepared starting from corresponding amino acid by introducing carboxy protecting group according to the procedure described in Protection for the Carboxyl Group. Kohlbau, H. J.; Thtirmer, R.; Voelter, W; In Synthesis of Peptides and Peptidomimetics; M. Goodman, Ed., Houben-Weyl, 4th ed., Vol. E22a; Thieme Stuttgard, 2002, pp 193 - 259.

Compound of formula (V) may also be prepared through esterification reaction from corresponding acid and alcohol in the presence of acidic catalyst (Noboru Ieda at al., Ind. Eng. Chem. Res. 2008, 47, 8631-8638; Naowara Al-Arafi at al., E-Journal of Chemistry 2012, 9(1), 99-106; Mantri, K. Chem. Lett. 34 (2005) 11, 1502-1503).

Alternatively, compounds of Formula (I) wherein A is -(CH₂)ₘ-CH₃ and B is -NH-(CH₂)ₙ-CO-R² and R² is -OR³, may be prepared by reaction of hydrochloride salt of compound of formula (V)

NH₂(CH₂)ₙCOOR³ (V)

and compound of formula (VI)

CH₃-(CH₂)ₘ-CO-NH-CH(R¹)-COOH (VI)

under activation by Ph₃P, in CCl₄/MeCN as solvent in the presence of Et₃N.

Compound of formula (VI) may be prepared starting with ester of formula

CH₃-(CH₂)ₘ-CO-NH-CH(R¹)-CO-OCH₃ (VIa)

by saponification with NaOH in methanol solution followed by acidification.

Compounds of Formula (VIa) may be prepared from ester of formula (VII)

CH₃-(CH₂)ₘ-COOSu (VII)

with the compound of formula (VIII)

HCl x NH₂-CH(R¹)-COOCH₃ (VIII)

in dry dioxane in the presence of Et₃N.

Compound of formula (VII) may be prepared starting from acid of formula (VIIa)

CH₃-(CH₂)ₘ-COOH (VIIa)

by reaction with N-hydroxy-succinimide in the presence of DCC in dry dioxane.

Compound of formula (I) wherein A is -CO-NH-CH(R¹)-CO-CH₃ and B is R² wherein R² is -NH₂ may be prepared starting from corresponding compound of formula (I) wherein R² is - OCH₃ by amonolysis in conc. NH₃/MeOH for 5-10 days at 0 °C.

Compound of formula (I) wherein A is -CO-NH-CH(R¹)-CO-CH₃ and B is R² wherein R² is -OR³ may be prepared by condensation of oxalylchloride of formula (COCl)₂ with the salt of corresponding amino acid ester of formula (VIII)

NH₂-CH(R¹)-COOR³ (VIII)

in aprotic solvent such as dichloromethane in the presence of Et₃N at 0 °C to room temperature overnight.

Compound of formula (VIII) are commercially available in case R³ is Me, tert-buthyl or benzyl, or are easily prepared starting from corresponding amino acid by introducing carboxy protecting group according to the procedure described in Protection for the Carboxyl Group. Kohlbau, H. J.; Thtirmer, R.; Voelter, W; In Synthesis of Peptides and Peptidomimetics; M. Goodman, Ed., Houben-Weyl, 4th ed., Vol. E22a; Thieme Stuttgard, 2002, pp 193 - 259.

Compound of formula (VIII) may also be prepared with condensation reaction from corresponding amino acid and alcohol in benzene in presence of acidic catalyst under reflux in a Dean-Stark apparatus.

Compound of formula (I) wherein A is -CO-NH-CH(R¹)-CO-CH₃ and B is R² wherein R² is - OH may be prepared by condensation of oxalylchloride of formula (COCl)₂ with the corresponding amino acid of formula (IV)

NH₂-CH(R¹)-COOH (IV).

The reaction is carried out in biphasic CH₂Cl₂ / aqueous KOH system by adding dropwise a solution of oxalyl chloride in CH₂Cl₂ and aqueous KOH to a cooled (-10 °C) solution of the corresponding amino acid continuing with stirring at 0°C to a room temperature for 1 hour. The product precipitates from aqueous layer after diluting with H₂O, and acidifying with formic acid.

Compound of formula (I) wherein A is -CO-NH-(CH₂)ₚ-NH-CO-CO-NH-CH(R¹)-CO-R² and B is R² wherein R² is -OH may be prepared by alkaline hydrolysis (e.g. with a LiOH) of corresponding compound wherein R² is -OMe in an aprotic solvent such as dichloromethane at room temperature.

Compound of formula (I) wherein A is -CO-NH-(CH₂)ₚ-NH-CO-CO-NH-CH(R¹)-CO-R² and B is R² wherein R² is -OR³ may be prepared by transforming diacid of formula (IX)

HOOC-CO-NH-(CH₂)ₚ-NH-CO-COOH (IX)

first to corresponding dichlorides with SOCl₂ in the presence of catalytic amount of DMF in dichloromethane as solvent, followed by reaction with salt of corresponding amino acid ester of formula (VIII)

NH₂-CH(R¹)-COOR³ (VIII)

in the presence of Et₃N.

Compound of formula (IX) may be prepared by treating the diester of formula (X)

EtOOC-CO-NH-(CH₂)ₚ-NH-CO-COOEt (X)

with methanolic KOH followed by acidification.

Diester of formula (X) may be prepared by condensation of diamine of formula (XI)

H₂N-(CH₂)ₚ-NH₂ (XI)

with ethyl oxalylchloride of formula EtO-CO-COCl in the presence of Et₃N in the aprotic solvent such as dichloromethane.

Compounds of formula (II), (IV), (V), (VIIa) (VIII) and (XI) are commercially available or are easily prepared by person skilled in the art.

### Examples

**Example 1:** 6-{[2-(heptadecanoylamino)-2-phenylethanoyl]amino}hexanoic acid
**Example 2:** 4-{[2-(nonadecanoylamino)-2-phenylethanoyl]amino}butanoic acid
**Example 3:** 4-{[(2*R*)-2-(nonadecanoylamino)-2-phenylethanoyl]amino}butanoic acid
**Example 4:** Sodium 4-{[2-(nonadecanoylamino)-2-phenylethanoyl]amino}butanoate
**Example 5:** 2-{[2-(heneicosanoylamino)-2-phenylethanoyl]amino}acetic acid
**Example 6:** 2-1[(2*R*)-2-(hencicosanoylamino)-2-phenylethanoyl]amino}acetic acid
**Example 7:** 11-{[(2*S*)-2-(Dodecanoylamino)-3-methylbutanoyl]amino}undecanoic acid
**Example 8:** 11-{[(2*R*,*S*)-2-(Dodecanoylamino)-3-methylbutanoyl]amino}undecanoic acid
**Example 9:** 11-{[(2*S*)-2-(Dodecanoylamino)-3-phenylpropanoyl]amino}undecanoic acid
**Example 10:** 11-[(2*R*,*S*)-2-(Dodecanoylamino)-3-phenylpropanoyl]amino}undecanoic acid
**Example 11:** 11-{[(2*R*,*S*)-2-(Dodecanoylamino)-4-methylpentanoyl]amino}undecanoic acid

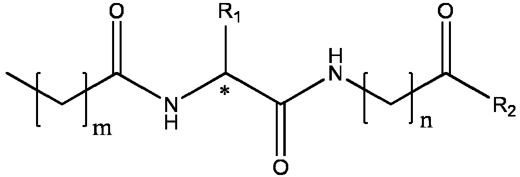

| **Example** | **R¹** | **R²** | **m** | **n** | **stereochemistry** |
|---|---|---|---|---|---|
| **1** | -Ph | -OH | 15 | 5 | *rac* |
| **2** | -Ph | -OH | 17 | 3 | *rac* |
| **3** | -Ph | -OH | 17 | 3 | *R* |
| **4** | -Ph | -O⁻Na⁺ | 17 | 3 | *rac* |
| **5** | -Ph | -OH | 19 | 1 | *rac* |
| **6** | -Ph | -OH | 19 | 1 | *R* |
| **7** | -CH(CH₃)₂ | -OH | 10 | 10 | *S* |
| **8** | -CH(CH₃)₂ | -OH | 10 | 10 | *rac* |
| **9** | -CH₂Ph | -OH | 10 | 10 | *S* |
| **10** | -CH₂Ph | -OH | 10 | 10 | *rac* |
| **11** | -CH₂CH(CH₃)₂ | -OH | 10 | 10 | *rac* |

Compounds of Examples 1-6 were synthesised according to the procedure described in Čaplar at al., *Chem. Eur. J.* 2010, 16, 3066 - 3082.

Compounds of Examples 7-11 were synthesised according to the procedure described in Čaplar at al., *Eur. J. Org. Chem.* 2004, 4048-4059.
**Example 12:** *N*,*N'*-Oxalyl-bis((*S*)-leucylamide)
**Example 13:** *N*,*N'*-Oxalyl-bis((*S*)-leucine methyl ester)
**Example 14:** *N*,*N'*-Oxalyl-bis((*S*)-valylamide)
**Example 15:** *N*,*N'*-Oxalyl-bis((*S*)-ValOH)
**Example 16:** *N*,*N'*-Oxalyl-bis((*S*)-phenylalanylamide)
**Example 17:** *N*,*N'*-Oxalyl-bis((*S*)-PheOH)
**Example 18:** *N*,*N'*-Oxalyl-bis[(*S*)-phenylalanine methyl ester)]
**Example 19:** (±)-[N,N'-Oxalyl-bis(phenylglycylamide)]
**Example 20:** N,N'-Oxalyl-bis((R)-PhgOH)
**Example 21:** *N*,*N'*-Oxalyl-bis[(*R*)-phenylglycine methyl ester)]

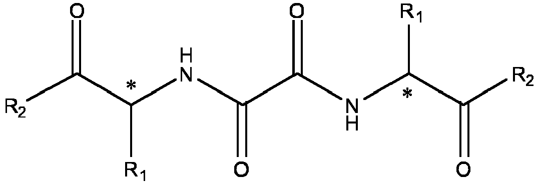

| **Example** | **R¹** | **R²** | **stereochemistry** |
|---|---|---|---|
| **12** | -CH₂CH(CH₃)₂ | -NH₂ | *S*,*S* |
| **13** | -CH₂CH(CH₃)₂ | -OCH₃ | *S*,*S* |
| **14** | -CH(CH₃)₂ | -NH₂ | *S*,*S* |
| **15** | -CH(CH₃)₂ | -OH | *S*,*S* |
| **16** | -CH₂Ph | -NH₂ | *S*,*S* |
| **17** | -CH₂Ph | -OH | *S*,*S* |
| **18** | -CH₂Ph | -OCH₃ | *S*,*S* |
| **19** | -Ph | -NH₂ | *rac* |
| **20** | -Ph | -OH | *R,R* |
| **21** | -Ph | -OMe | *R,R* |

Compounds of Examples 12-21 were synthesised according to the procedure described in Makarević at al., *Chem. Eur. J.* 2001, 7 (15), 3328 - 3341.
**Example 22:** 1,6-Bis((O-leucylmethanol)-*N*-yloxalamido)hexane
**Example 23:** 1,6-Bis ((leucine)-*N*-yloxalamido)hexane
**Example 24:** 1,9-Bis((O-leucylmethanol)-*N*-yloxalamido)nonane
**Example 25:** 1,9-Bis ((leucine)-*N*-yloxalamido)nonane

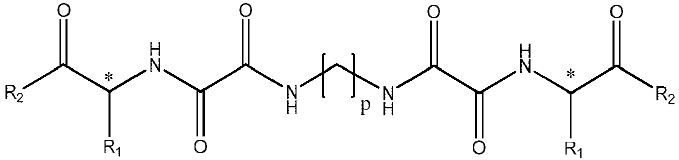

| **Example** | **R¹** | **R²** | **p** | **stereochemistry** |
|---|---|---|---|---|
| **22** | -CH₂CH(CH₃)₂ | -OCH₃ | 6 | *S*,*S* |
| **23** | -CH₂CH(CH₃)₂ | -OH | 6 | *S*,*S* |
| **24** | -CH₂CH(CH₃)₂ | -OCH₃ | 9 | *S*,*S* |
| **25** | -CH₂CH(CH₃)₂ | -OH | 9 | *S*,*S* |

Compounds of Examples 22-25 were synthesised according to the procedure described in Šijaković Vujičić at al. *Chem. Eur. J.* 2013, 19, 8558 - 8572.

The procedures and spectroscopic data of Examples 1-25 are incorporated here by references.

### Determination of Gelling Properties:

All gelation experiments were performed in test tubes of 12 mm in diameter.

The tested substance was placed in a test tube, and the oil was added by micro syringe in 500 µL portions. After each addition the mixture was gently heated until the substance dissolved, and was then allowed to cool spontaneously to room temperature and formation of gel checked by test tube inversion. The procedure is repeated until formation of a loose gel or dissolution is observed.

Gelation properties of prepared compounds were tested against various edible oils and the results are collected in Tables 1 and 2. Gelation efficiency of each gelator toward the specified edible oil is expressed in mL of oil that could be immobilized by 10 mg of the gelator.

All of the prepared gels are transparent and show thermoreversible gel-to-sol transitions.

In experiments sunflower oil (Zvijezda d.o.o.), soybean oil (Fluka), olive oil (Primadonna) were used.

### Determination of thixotropic property

The formed gel was subjected to external mechanical stress (shaking) till gel is transformed into a sol state. The self-healing process (recovery to gel-state) after standing at room temperature was measured every 5 minutes by tube inversion method and visual observation.

The time of response necessary to self-heal from sol to gel state was determined at a half of the maximum gelling volume per 10 mg of tested compound (Table 1, column Recovery time (h or min)).

For example, compound **12** forms a transparent gel after a heating-cooling process in soybean oil with a critical gelation concentration (CGC) of 0.025 wt% (which corresponds to 43.3 mL expressed as maximal volume of oil that could be immobilized by 10 mg of compound **12,** see Table 1). When treated with external mechanical stress, gel **12** from soybean oil lost most of its viscosity and transformed into a sol; after resting for 5 min at room temperature, the gel completely regenerated. The self-healing process after the gel to sol transition, which was brought about by mechanical stress, can be repeated many times.

**Table 1. Gelation efficiency of compounds 1, 2, 3, 6, 7, 9, 10, 12, 15, 16, 22 and 24 toward the specified edible oil expressed as the maximal volume (Vₘₐₓ/ml) of oil that could be immobilized by 10 mg of the gelator. Thixotropic property measured at a half of the maximum gelling volume.**

| **Compound** | **Sunflower oil** V / ml | Recovery time* / h or min | **Soybean oil** V/ml | Recovery time / h or min | **Olive oil** V/ml | Recovery time / h or min |
|---|---|---|---|---|---|---|
| **1** | **9.5** | no | **5.8** | no | **1.8** | no |
| **2** | **9.8** | 12 h | **12** | 20 min | **26.3** | 35 min |
| **3** | **12.7** | no | **18** | no | **37.2** | no |
| **6** | **7.2** | 10 min | **13.7** | 10 min | **5.7** | 5 min |
| **7** | **15.3** | 5 min | **18.7** | 35 min | **18.5** | 10 min |
| **9** | **14** | 1.5 h | **11.1** | 2.5 h | **21.8** | 4h |
| **10** | **13.4** | 12 h | **2** | no | **1.8** | 72 h |
| **12** | **13.2** | 5 min | **43.3** | 5 min | **27.2** | 10 min |
| **15** | **9.4** | 4h | **14.5** | 5 min | **15.2** | 5 min |
| **16** | **24.5** | no | **32** | no | **15.2** | no |
| **22** | **17.3** | 5 min | **16.6** | 5 min | **25.9** | 30 min |
| **24** | **9** | 25 min | **7** | 5 min | **8.7** | 15 min |

| | | | | | | |
|---|---|---|---|---|---|---|
| ***Self-healing properties of gels were checked by test-tube inversion every 5 minutes** | | | | | | |

**Table 2. Gelation efficiency of compounds 4, 5, 8, 11, 13, 14, 17 - 21, 23 and 25 expressed as the maximal volume (Vₘₐₓ/ ml) of sunflower oil that could be immobilized by 10 mg of the gelator.**

| **Compound** | **4** | **5** | **8** | **11** | **13** | **14** | **17** | **18** | **19** | **20** | **21** | **23** | **25** |
|---|---|---|---|---|---|---|---|---|---|---|---|---|---|
| **V/mL** | 1.9 | NG | 3.5 | NG | NG | 3 | 1.8 | 1.8 | NG | NG | 2 | NG | 5** |

| | | | | | | | | | | | | | |
|---|---|---|---|---|---|---|---|---|---|---|---|---|---|
| *NG= no gelation; **very weak gelation after 24 h | | | | | | | | | | | | | |

## Claims

1. The composition with gelling properties comprising a compound of Formula (I) or a salt thereof 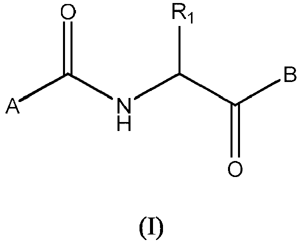 and a vegetable oil;
wherein A is selected from:
i) -(CH₂)ₘ-CH₃,
ii) -CO-NH-CH(R¹)-CO-CH₃ and
iii) -CO-NH-(CH₂)ₚ-NH-CO-CO-NH-CH(R¹)-CO-R²
B is selected from:
i) -NH-(CH₂)ₙ-CO-R² and
ii) -R².
R¹ is H, C₁-C₄ alkyl, phenyl, or -CH₂Ph;
R² is -OH, -NH₂ or -OR³;
R³ is C₁-C₄ alkyl or -CH₂Ph;
m is an integer from 1-34;
and n is an integer from 1-22;
p is an integer from 1-12;
provided that when A is -(CH₂)ₘ-CH₃ then B is -NH-(CH₂)ₙ-CO-R²
and when B is -NH-(CH₂)ₙ-CO-R² then A is -(CH₂)ₘ-CH₃.

2. A composition according to claim 1 wherein the composition is in the form of gel.

3. A composition according to claim 1 or 2 wherein the oil is edible oil selected from palm oil, sunflower oil, olive oil, soybean oil, linseed oil, rapeseed oil, corn oil, pumpkin seed oil, sesame oil, safflower oil, castor, peanut oil or combination thereof.

4. A composition according to claim 1 or 2 wherein the oil is base cosmetic oil selected from sweet almond oil, linseed oil, grape seed oil, avocado oil, apricot oil, olive oil, sesame oil, rapeseed oil, sunflower oil, jojoba oil, castor oil, borage seed oil, argan oil, avocado oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil or combination thereof.

5. A composition according to claim 1 or 2 wherein the oil is selected from sunflower oil, olive oil, soybean oil or combination thereof.

6. A composition according to any of claims 1 to 5 wherein A is -(CH₂)ₘ-CH₃ and B is -NH-(CH₂)ₙ-CO-OR².

7. A composition according to claim 6 wherein sum of n and *m* is between 18 and 22.

8. A composition according to any of claims 1 to 5 wherein A is -CO-NH-CH(R¹)-CO-CH₃ and B is R².

9. A composition according to any of claims 1 to 5 wherein A is -CO-NH-(CH₂)ₚ-NH-CO-CO-NH-CH(R¹)-CO-R² and B is R².

10. A composition according to any of claims 6 to 9 wherein R² is -OH, NH₂ or -OCH₃.

11. A composition according to any of claims 6 to 10 wherein R¹ is -CH₂Ph, Ph, - CH₂CH(CH₃)₂ or -CH(CH₃)₂.

12. A composition according to any of claims 1 to 11 further comprising water.

13. A composition according to any of claims 1 to 12 further comprising either:
(a) a food;
(b) a cosmetically acceptable ingredient or
(c) a pharmaceutically acceptable ingredient.

14. A composition according to any of claims 1 to 12 wherein the compound of formula (I) or a salt thereof is present in the composition in the concentration of about 0.02 to about 10 wt % relative to the total weight of the composition.

15. A process for the preparation of a composition according to the previous claims comprising the following steps:
(a) mixing the compounds of formula (I) or a salt thereof and the oil;
(b) heating the mixture obtained in step (a) to a temperature until compound of formula (I) is completely dissolved;
optionally (c) mixing the food, pharmaceutically active ingredient, nutraceuticals or a cosmetic ingredient as solid or solubilised component in oil or water into the mixture during the step (b);
(d) cooling the mixture obtained in step (b) to room temperature or below.

## Patentansprüche

1. Ein Präparat mit Geliereigenschaften, das eine Verbindung der Formel (I) oder ein Salz davon 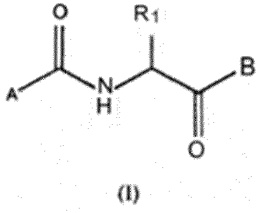 und Pflanzenöl enthält;
wobei A ausgewählt ist unter:
i) -(CH₂)ₘ-CH₃,
ii) -CO-NH-CH(R¹)-CO-CH₃ und
iii) -CO-NH-(CH₂)ₚ-NH-CO-CO-NH-CH(R₁)-CO-R²
B ausgewählt ist unter:
i) -NH-(CH₂)ₙ-CO-R² und
ii) -R².
R¹ ist H, C₁-C₄-Alkyl, -Phenyl oder -CH₂Ph;
R² ist -OH, -NH₂ oder -OR³;
R³ ist C₁-C₄-Alkyl oder -CH₂Ph;
m ist eine ganze Zahl von 1 bis 34;
und n ist eine ganze Zahl von 1 bis 22;
p ist eine ganze Zahl von 1 bis 12;
unter der Bedingung, wenn A ist -(CH₂)ₘ-CH₃, dann B ist -NH-(CH₂)ₙ-CO-R² und wenn B ist -NH-(CH₂)ₙ-CO-R², dann A ist -(CH₂)ₘ-CH₃

2. Präparat nach Anspruch 1, **gekennzeichnet dadurch, dass** das Präparat in Gelform ist.

3. Präparat nach Anspruch 1 oder 2, **gekennzeichnet dadurch, dass** das Öl ein aus Palmöl, Sonnenblumenöl, Olivenöl, Sojaöl, Leinöl, Rapsöl, Maisöl, Kürbiskernöl, Sesamöl, Distelöl, Rizinusöl, Erdnussöl oder einer Kombination davon ausgewähltes Speiseöl ist.

4. Präparat nach Anspruch 1 oder 2, **gekennzeichnet dadurch, dass** das Öl ein aus Süßmandelöl, Leinöl, Traubenkernöl, Avocadoöl, Aprikosenöl, Olivenöl, Sesamöl, Rapsöl, Sonnenblumenöl, Jojobaöl, Rizinusöl, Borretschsamenöl, Arganöl, Avocadoöl, Ringelblumenöl, Nachtkerzenöl, Haselnussöl, Walnussöl, Erdnussöl, Macadamiaöl, Kokosöl, Hagebuttenkernöl, Weizenkeimöl, Johanniskrautöl, Heidelbeersamenöl, Schwarzkümmelsamenöl, Reiskleieöl oder eine Kombination davon ausgewähltes kosmetisches Basisöl ist.

5. Präparat nach Anspruch 1 oder 2, **gekennzeichnet dadurch, dass** das Öl aus Sonnenblumenöl, Olivenöl, Sojaöl oder einer Kombination davon ausgewählt ist.

6. Präparat nach einem beliebigen Patentanspruch 1 bis 5, **gekennzeichnet dadurch, dass** A ist -(CH₂)ₘ-CH₃ und B ist -NH-(CH₂)ₙ-CO-OR².

7. Präparat nach Anspruch 6, **gekennzeichnet dadurch, dass** die Summe von *n* und *m* zwischen 18 und 22 beträgt.

8. Präparat nach einem beliebigen Patentanspruch 1 bis 5, **gekennzeichnet dadurch, dass** A ist -CO-NH-CH(R¹)-CO-CH₃ und B ist R².

9. Präparat nach einem beliebigen Patentanspruch 1 bis 5, **gekennzeichnet dadurch, dass** A ist -CO-NH-(CH₂)ₚ-NH-CO-CO-NH-CH(R¹)-CO-R² und B ist R².

10. Präparat nach einem beliebigen Patentanspruch 6 bis 9, **gekennzeichnet dadurch, dass** R² ist -OH, NH₂ oder -OCH₃.

11. Präparat nach einem beliebigen Patentanspruch 6 bis 10, **gekennzeichnet dadurch, dass** R¹ ist -CH₂Ph, Ph, - CH₂CH(CH₃)₂ oder -CH(CH₃)₂

12. Präparat nach einem beliebigen Patentanspruch 1 bis 11, **gekennzeichnet dadurch, dass** es zudem Wasser enthält.

13. Präparat nach einem beliebigen Patentanspruch 1 bis 12, **gekennzeichnet dadurch, dass** zudem enthält entweder:
(a) Lebensmittel;
(b) kosmetisch akzeptabler Inhaltsstoff oder
(c) pharmazeutisch akzeptabler Inhaltsstoff.

14. Präparat nach einem beliebigen Patentanspruch 1 bis 12, **gekennzeichnet dadurch, dass** die Verbindung der Formel (I) oder ein Salz davon in der Zubereitung in einer Konzentration von etwa 0,02 bis etwa 10 Gew.-% des Gesamtgewichts des Präparats vorhanden ist.

15. Verfahren zur Zubereitung des Präparats nach dem Anspruch umfassend folgende Schritte:
(a) Mischen von Verbindungen der Formel (I) oder Salze davon in das Öl;
(b) Erhitzen der in Schritt (a) erhaltenen Mischung auf eine Temperatur, bis die Verbindung der Formel (I) vollständig gelöst ist;
optional (c) Mischen des Lebensmittels, pharmazeutischen Wirkstoffs, Nutrazeutikums oder kosmetischen Inhaltsstoffs als feste oder in Öl oder Wasser gelöste Komponente in die Mischung während Schritt (b);
(d) Abkühlen der in Schritt (b) erhaltenen Mischung auf Raumtemperatur oder niedriger.

## Revendications

1. Composition aux propriétés gélifiantes comprenant un composé de formule (I) ou son sel 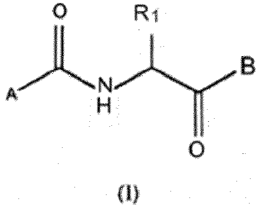 et une huile végétale ;
dans laquelle A est choisi parmi :
i)-(CH₂)ₘ-CH₃,
ii) ii) -CO-NH-CH(R1 )-CO-CH3 et,
iii) -CO-NH-(CH₂)ₚ-NH-CO-CO-NH-CH(R¹)-CO-R²
B est choisi parmi :
i) -NH-(CH₂)n-CO- R² et
ii) - R²
R¹ est H, C₁-C₄ alkyle, phényle, ou -CH₂Ph ;
R² est -OH, -NH₂ ou -OR³ ;
R³ est C₁-C₄ alkyle ou -CH₂Ph ;
m est un nombre entier de 1 à 34 ;
et n est un nombre entier de 1 à 22 ;
p est un nombre entier de 1 à 12 ;
à condition que lorsque A est -(CH₂)ₘ-CH₃ puis B est -NH-(CH₂)ₙ-CO-R²
et quand B est -NH-(CH₂)ₙ-CO-R² puis A est -(CH₂)m-CH₃.

2. Composition selon la revendication 1, dans laquelle la composition est sous forme de gel.

3. Composition selon la revendication 1 ou 2, dans laquelle l'huile est une huile comestible choisie parmi l'huile de palme, l'huile de tournesol, l'huile d'olive, l'huile de soja, l'huile de lin, l'huile de colza, l'huile de maïs, l'huile de graines de citrouille, l'huile de sésame, l'huile de carthame, l'huile de ricin, l'huile d'arachide, huile ou une combinaison de celles-ci.

4. Composition selon la revendication 1 ou 2, dans laquelle l'huile est une huile cosmétique de base choisie parmi l'huile d'amande douce, l'huile de lin, l'huile de pépins de raisin, l'huile d'avocat, l'huile d'abricot, l'huile d'olive, l'huile de sésame, l'huile de colza, l'huile de tournesol, l'huile de jojoba, l'huile de ricin, huile de bourrache, huile d'argan, huile d'avocat, huile de calendula, huile d'onagre, huile de noisette, huile de noix, huile d'arachide, huile de macadamia, huile de coco, huile de graines d'églantier, huile de germe de blé, huile de millepertuis, myrtille huile de graines, graines de cumin noir, huile de son de riz ou leur combinaison.

5. Composition selon la revendication 1 ou 2, dans laquelle l'huile est choisie parmi l'huile de tournesol, l'huile d'olive, donc l'huile de haricot ou de leur combinaison.

6. Composition selon l'une quelconque des revendications 1 à 5, dans laquelle A est -(CH₂)ₘ-CH₃ et B est -NH-(CH₂)n-CO-OR².

7. Composition selon la revendication 6, dans laquelle la somme de *n* et *m* est comprise entre 18 et 22.

8. Composition selon l'une quelconque des revendications 1 à 5, dans laquelle A représente -CO-NH-CH(R¹)-CO-CH₃ et B est R².

9. Composition selon l'une quelconque des revendications 1 à 5, dans laquelle A est -CO-NH-(CH₂)p-NH-COCO-NH-CH(R1)-CO-R² et B est R².

10. Composition selon l'une quelconque des revendications 6 à 9, dans laquelle R² est -OH, NH₂ ou -OCH₃.

11. Composition selon l'une quelconque des revendications 6 à 10, dans laquelle R¹ est -CH₂Ph, Ph, - CH₂CH(CH₃)₂ ou -CH(CH₃)₂.

12. Composition selon l'une quelconque des revendications 1 à 11, comprenant en outre de l'eau.

13. Composition selon l'une quelconque des revendications 1 à 12, comprenant en outre soit :
(a) un aliment ;
(b) un ingrédient cosmétique acceptable où
(c) un ingrédient pharmaceutique acceptable.

14. Composition selon l'une quelconque des revendications 1 à 12, dans laquelle le composé de formule (I) ou un sel de celui-ci est présent dans la composition à une concentration d'environ 0,02 à environ 10 % en poids par rapport au poids total de la composition

15. Procédé pour la composition de préparation selon la revendication comprenant les étapes suivantes :
(a) mélanger les composés de formule (I) ou un son sel et l'huile ;
(b) chauffer le mélange obtenu à l'étape (a) à une température jusqu'à ce que le composé soit complètement dissous ; facultativement (c) mélanger l'aliment, l'ingrédient pharmaceutique actif, les produits nutraceutiques ou un ingrédient cosmétique en tant que composant solide ou solubilisé dans de l'huile ou de l'eau dans le mélange au cours de l'étape (b) ;
(d) refroidir le mélange obtenu à l'étape (b) à température ambiante ou en dessous.
